(19) **Europäisches Patentamt / European Patent Office / Office européen des brevets**

(11) **EP 4 767 933 A1**

(12) **EUROPEAN PATENT APPLICATION**
published in accordance with Art. 153(4) EPC

(43) Date of publication:
01.07.2026 Bulletin 2026/27

(21) Application number: 24858821.2

(22) Date of filing: 23.08.2024

(51) International Patent Classification (IPC):
$A61B\ 5/145^{(2006.01)}$     $A61M\ 5/142^{(2006.01)}$
$A61M\ 5/172^{(2006.01)}$     $G16H\ 10/60^{(2018.01)}$
$G16H\ 40/60^{(2018.01)}$

(86) International application number:
PCT/ES2024/070517

(87) International publication number:
WO 2025/046158 (06.03.2025 Gazette 2025/10)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 25.08.2023 ES 202330715

(71) Applicant: Universitat Politècnica de València
46022 Valencia (ES)

(72) Inventors:
• SANZ DÍAZ, Ricardo
46022 Valencia (ES)
• BONDÍA COMPANY, Jorge
46022 Valencia (ES)
• DIEZ RUANO, José Luís
46022 Valencia (ES)
• GARCÍA GIL, Pedro José
46022 Valencia (ES)
• SALA MIRA, Iván
46022 Valencia (ES)

(74) Representative: Pons IP
Glorieta Rubén Darío 4
28010 Madrid (ES)

(54) **METHOD FOR CONTROLLING BLOOD GLUCOSE AND ARTIFICIAL PANCREAS SYSTEM FOR CARRYING OUT THE METHOD**

(57)     A method for controlling glucose in a flexibly structured dual-hormone artificial pancreas that manages optional meal and/or exercise notifications using coordinated control actions, which comprises: measuring a plasma glucose signal; calculating an incremental plasma glucose measurement (y); defining a model for incremental plasma glucose; defining a carbohydrate intake as dependent on a carbohydrate content estimated by the patient; defining an expected postprandial incremental plasma glucose y*(s) depending on the insulin bolus administered; defining a corrected incremental plasma glucose $\overline{y}(s)$, a corrected insulin infusion $\overline{u}(s)$, and a corrected carbohydrate $\overline{d}(s)$ intake; defining a virtual control action $\mu(s)$, divided into regulatory actions $\mu_r$ and counter-regulatory actions $\mu_{cr}$ and calculating control actions using a 2-DOF feedback controller with a nominal value pre-filter $F_r(s)$.

EP 4 767 933 A1

**Description**

## OBJECT OF THE INVENTION

The present invention is comprised in the technological field of glucose control. Specifically, the invention relates to regulatory control actions by means of the administration of insulin and counter-regulatory control actions by means of the administration of glucagon and/or rescue carbohydrates, to control glucose levels more effectively.

An object of the present invention is to provide a method for controlling glucose that is capable of managing optional meal and exercise notifications by means of a flexibly structured dual-hormone control system.

A second object of the present invention is to provide a flexibly structured dual-hormone artificial pancreas for the administration of insulin, glucagon and/or rescue carbohydrates in a coordinated manner.

## BACKGROUND OF THE INVENTION

Type 1 diabetes *mellitus* (T1DM) is an autoimmune disorder that destroys pancreatic β-cells, resulting in the inability to secrete insulin. This hormone plays a crucial role in glucose homeostasis, since it is responsible for reducing plasma glucose concentration.

As a result, people with T1DM tend to have high blood glucose levels for prolonged periods of time (hyperglycaemia), giving rise to serious long-term health problems, such as cardiovascular diseases, nephropathy, retinopathy, and neuropathy. This is why exogenous insulin administration is needed.

Artificial pancreas (AP) systems have emerged as a technological treatment for T1DM, improving blood glucose control over traditional treatments. However, daytime control remains a challenge because meal intake and exercise cause substantial fluctuations in glucose levels. Current hybrid AP systems are based on prandial boluses with carbohydrates adapted to compensate for increased glucose due to meals. In open-loop treatments, patients already have to deal with the responsibility of estimating carbohydrates and notifying the system of same (meal notification).

Therefore, some patients may not perceive the usefulness of AP, discouraging its continuation. Hence, an AP system must be able have acceptable performance without the responsibility of meal notifications. However, a complete system without meal notification (fully automated) may not suit all patients. Some patients with extensive experience in estimating carbohydrates may prefer to take on, at least on some occasions, the responsibility of meal notification to reinforce postprandial control.

However, meal notifications, as a feedforward action, may interact with the feedback action in an AP designed to work in a fully automated manner without said notification, giving rise to an excessive administration of insulin, and as a result, causing hypoglycaemia (abnormally low glucose levels). Therefore, AP systems must incorporate mechanisms that allow the user to provide meal notifications without increasing the risk of hypoglycaemia.

Exercise is the other main problem challenging the performance of the AP system. Although very intense exercise events may result in hyperglycaemia (abnormally high glucose levels), mild to moderate aerobic exercise, the most common physical activity practised by non-athletes, lowers glucose levels. Current hybrid systems require users to plan exercise to reduce insulin infusion hours before the start of exercise.

To eliminate exercise notifications, the AP system must have counter-regulatory control actions that raise glucose levels. Taking carbohydrate supplementation is the most practical option for treating mild exercise-related hypoglycaemia. However, people concerned about weight gain may prefer subcutaneous administration of glucagon as a non-caloric treatment for hypoglycaemia. Dual-hormone AP systems, with the automatic administration of insulin and glucagon, have effectively reduced the time of exercise-induced hypoglycaemia in clinical trials.

People who are less physically active and have a low tendency to hypoglycaemia may find the additional cost of dual-hormone AP systems for the timely administration of glucagon unjustifiable. Self-administered low-dose pen glucagon may be a more suitable option for these patients for safe and effective mitigation of non-severe hypoglycaemia.

Based on the foregoing analysis, the following three characteristic features are recommendable in an AP system:

1) effectively compensating for meals without any meal notification,

2) allowing patients to provide meal notifications if they wish to do so without compromising the performance of the feedback controller, and

3) offering a counter-regulatory control action to manage hypoglycaemia mitigation, such as in non-notified exercise events.

Many methods have been proposed to eliminate meal notifications, but none have mechanisms to reduce interactions with the feedback action in the event that the patient provides meal notifications.

With respect to feedforward as a result of unplanned exercise, most glucagon-insulin systems lack carbohydrate-suggesting modules and vice versa.

The few strategies that may work with glucagon and carbohydrates to mitigate hypoglycaemia require meal notifications.

However, as explained, a coordinated dual-hormone AP system without meal notifications that allows patients to provide meal notifications if they prefer to do so and to decide on the counter-regulatory action between glucagon and carbohydrates has not been identified in the art.

## DESCRIPTION OF THE INVENTION

The method of the invention is a method for controlling blood glucose for a flexibly structured dual-hormone artificial pancreas. The method allows dealing with optional meal notifications and optional exercise notifications by means of using a coordinated control action.

The system coordinates a regulatory control action (insulin) and a counter-regulatory control action (glucagon and/or carbohydrates).

As explained above, the method of the invention manages non-notified meals, although the user can provide meal notifications if desired as a result of a non-interactive feedback-feedforward scheme that reduces interactions between feedback control and feedforward control.

In addition to insulin, the system provides a counter-regulatory control action to cope with induced hypoglycaemic situations, for example, due to non-notified exercise events. Users can choose whether they wish to administer this counter-regulatory action as an automatic infusion of glucagon by means of a pump, suggestions for glucagon administered by the patient with a pen, or suggestions for carbohydrate intake.

The method of the invention, therefore, is able to manage meals (non-notified or notified depending on the user's preference) and non-notified exercise events, offering in *in silico* studies parameters that are within the ranges recommended in clinical guidelines. The most significant change in performance corresponds to whether the patient decides to provide meal notifications or not. As expected, when providing meal notifications, the controller improves the % of time in hyperglycaemia of the operation without meal notification without noticeable degradation of hypoglycaemia-related parameters.

Furthermore, it has to be considered that, in *in silico* studies, there are no relevant differences in performance parameters between the use of glucagon or carbohydrates as a counter-regulatory action, since, by design, they provide the same control effort theoretically, with glucagon administration having the advantage of being a non-caloric supply and being able to be administered automatically by means of a pump.

The present invention allows providing near-optimal postprandial glucose control with and without meal or exercise notifications. Optimal is defined as a control method that minimises the blood glucose peak after a meal and, at the same time, prevents glucose values below a prescribed threshold under nominal conditions, in other words, a predefined hypoglycaemia limit (for example, 70 mg/dl).

A feedback controller provides a feedback control action that achieves near-optimal postprandial glucose control when meals are not notified. The optional meal notification is thus managed by means of a non-interactive feedback-feedforward scheme that inhibits insulin infusion after a pre-meal bolus of an optionally notified meal, preventing the excessive administration of insulin and maintaining performance.

The proposed solution defines a new control strategy designed to work with and without food or exercise notifications, achieving near-optimal postprandial glucose control under nominal conditions. The strategy includes pre-meal boluses in an insulin administration control designed to achieve near-optimal postprandial glucose in the absence of a meal notification, so as to achieve optimised coordination between a feedback control and feedforward actions. At the same time, it allows calculating whether a counter-regulatory control action is necessary, for example, due to exercise events.

It should be noted that the optimal open-loop policy consists of administering a bolus of insulin before mealtime, in other words, an impulsive feedforward control action, which cannot be generated by feedback since feedback control cannot be carried out until the output is affected by the disturbance. This leads to the conclusion that the best performance that can be achieved with feedback is obtained by administering a bolus of optimal size at the time when the output is affected by the disturbance.

The expected postprandial variation of the CGM ($y^*$), caused by the intake of meal notified by the patient and the associated bolus administered, is calculated on meal and insulin models. This expected trajectory is used to calculate a correction term for the control action provided by the feedback controller, preventing interactions between feedback and feedforward actions that may lead to excessive administration of insulin. As a result, the corrected feedback control action following a notified meal only regulates the deviation from the expected trajectory during the postprandial period due to the meal bolus.

Therefore, the method of the invention is designed for near-optimal blood glucose control with and without meal and/or exercise notifications and is configured as a near-optimal feedback controller designed to work without meal notifications and a coordination strategy between feedback-feedforward actions to adapt meal boluses from optionally notified meals. At the same time, the method of the invention allows calculating counter-regulatory control actions when necessary.

The method comprises the step of measuring a plasma glucose signal ($G(t)$) by means of a continuous glucose monitor (CGM), represented by its Laplace transform ($G(s)$). Hereinafter, any signal can be represented by its time domain (t) or Laplace domain (s), related by the Laplace transform. An incremental plasma glucose measurement ($y$) is introduced, which is calculated as:

$$y(t) = G(t) - G_b$$

wherein $G_b$ is a baseline glucose value resulting from the application of a baseline glucose infusion ($u_b$).

As such, an incremental insulin infusion is defined by:

$$u(t) = u_T(t) - u_b$$

wherein ($u_T$) is the total insulin infusion.

Furthermore, incremental plasma glucose ($y$) is defined taking into account carbohydrate intake ($d$), insulin infusion ($u$), a glucagon administration ($v$), and a rescue carbohydrate administration (w), defined by:

$$y(s) = G_u(s) \cdot u(s) + G_v(s) \cdot v(s) + G_w(s) \cdot w(s) + G_d(s) \cdot d(s)$$

wherein $u(t) \geq -u_b$, $v(t) \geq 0$ and $w(t) \geq 0$.

Carbohydrate administration (w) could be further processed by means of a quantifier to provide an amount that can be easily administered manually by the patient. Glucagon administration can also be quantified when easy manual administration by means of a pen is suggested to the patient. As an alternative, a quantified glucagon signal can also be automatically administered by means of a pump, instead of administering a continuous glucagon signal.

In the context of the invention $G_u$, $G_v$, $G_w$, $G_d$ are time-invariant linear transfer functions that correlate, respectively, insulin, glucagon, rescue carbohydrates, and carbohydrate intake from the meal, with glucose, and could be defined by means of multiple models known in the art, subject to the restriction that $G_u(s)/G_v(s)$ and $G_u(s)/G_w(s)$ must be feasible, in other words, the order of the numerator must be less than or equal to the order of the denominator.

Preferably, the models $G_u$, $G_v$, $G_w$, $G_d$ are defined by means of the following structure:

$$G_u(s) = \tilde{G}_u(s) \cdot e^{-l_u s}$$

$$G_v(s) = \tilde{G}_v(s) \cdot e^{-l_v s}$$

$$G_w(s) = \tilde{G}_w(s) \cdot e^{-l_w s}$$

$$G_d(s) = \tilde{G}_d(s) \cdot e^{-l_d s}$$

where $\tilde{G}_u(s)$, $\tilde{G}_v(s)$, $\tilde{G}_w(s)$ and $\tilde{G}_d(s)$ are transfer functions in the form:

$$\tilde{G}_u(s) = \frac{k_j^u}{(\tau_{1u} + 1)^2(\tau_{2u}s + 1)}$$

$$\tilde{G}_v(s) = \frac{k_j^v}{(\tau_{1v} + 1)^2(\tau_{2v}s + 1)}$$

$$\tilde{G}_w(s) = \frac{k_j^w}{(\tau_{1w} + 1)^2(\tau_{2w}s + 1)}$$

$$\tilde{G}_d(s) = \frac{k_j^d}{(\tau_{1d} + 1)^2(\tau_{2d}s + 1)}$$

wherein $l_u$, $l_v$, $l_w$ and $l_u$ are parameters representing delays, $k_j^u$, $k_j^v$, $k_j^w$, $k_j^d$, $\tau_{1u}$, $\tau_{2u}$, $\tau_{1v}$, $\tau_{2v}$, $\tau_{1w}$, $\tau_{2w}$, $\tau_{1d}$ and $\tau_{2d}$ are previously obtained parameters and j is an index representing a patient.

A carbohydrate intake is defined as:

$$d^*(s) = \begin{cases} M^* & notified\ meal \\ 0 & non - notified\ meal \end{cases}$$

with M* being an estimated carbohydrate content notified by a patient.

As such, an expected postprandial incremental plasma glucose (y*) is defined as:

$$y^*(s) = G_u(s) \cdot u^*(s) + G_d(s) \cdot d^*(s)$$

wherein $u^*(s)$ is the insulin bolus administered in association with the patient's manual meal notification.

Preferably, the insulin bolus $u^*(s)$ is calculated using a feedforward controller C(s) as:

$$u^*(s) = C(s) \cdot d^*(s)$$

As such, a corrected incremental plasma glucose $\bar{y}(s)$, a corrected insulin infusion $\bar{u}(s)$, and a corrected carbohydrate intake $\bar{d}(s)$ are defined as:

$$\bar{y}(s) = y(s) - y^*(s)$$

$$\bar{u}(s) = u(s) - u^*(s)$$

$$\bar{d}(s) = d(s) - d^*(s)$$

$$\bar{y}(s) = G_u(s) \cdot \bar{u}(s) + G_v(s) \cdot v(s) + G_w(s) \cdot w(s) + G_d(s) \cdot \bar{d}(s)$$

Using the corrected insulin infusion ($\bar{u}$), a virtual control action $\mu(s)$ can be defined as:

$$\mu(s) = \bar{u}(s) + \frac{G_v(s)}{G_u(s)} v(s) + \frac{G_w(s)}{G_u(s)} w(s)$$

such that

$$\bar{y}(s) = G_u(s) \cdot \mu(s) + G_d(s) \cdot \bar{d}(s)$$

This newly acquired virtual control action is then divided into a regulatory action ($\mu_r$) and a counter-regulatory action ($\mu_{cr}$):

$$\mu(t) \triangleq \mu_r(t) + \mu_{cr}(t)$$

further dividing the counter-regulatory action as

$$\mu_{cr}(s) \triangleq \mu_{cr,v}(s) + \mu_{cr,w}(s)$$

wherein $\mu_{cr,v}$ represents the counter-regulatory effect implemented by means of glucagon infusion and $\mu_{cr,w}$ represents the counter-regulatory effect implemented by means of carbohydrate suggestions, such that:

$$\mu_{cr,v}(s) \triangleq \frac{G_v(s)}{G_u(s)} v(s)$$

$$\mu_{cr,w}(s) \triangleq \frac{G_w(s)}{G_u(s)} w(s)$$

Regulatory and counter-regulatory actions are calculated as:

$$\mu_r(t) = \bar{u}(t) = \begin{cases} \mu_0(t) & if \ \sigma(t) \geq -\gamma u_b \\ -u_b & if \ \sigma(t) < -\gamma u_b \end{cases}$$

$$\mu_{cr}(t) = \begin{cases} 0 & if \ \sigma(t) \geq -\gamma u_b \\ k_{cr} \cdot \mu_0(t) + u_b & if \ \sigma(t) < -\gamma u_b \end{cases}$$

wherein $\sigma(t)$ is a function defining the change between regulatory and counter-regulatory actions (switching signal), for example $\sigma(t) = \mu_0(t)$ or $\mu_0(t)$ filtering such as a moving average filter with a specific sample window, and $\gamma$ is an adjustable factor, and wherein $k_{cr}$ is a previously obtained controller gain, and $\mu_0$ is defined as:

$$\mu_0(s) = \mu_{dob}(s)$$

with $\mu_{dob}$ being calculated as a 2-DOF feedback controller with a nominal value pre-filter $F_r(s)$ as:

$$\mu_{dob}(s) = K(s) \cdot \left( F_r(s) \cdot r(s) - \bar{y}(s) \right)$$

$$\mu_{dob}(s) = K(s) \cdot \left( F_r \cdot r(s) - y(s) \right) + K(s) \cdot y^*(s)$$

and wherein $K(s)$ is a central linear controller designed to stabilise the system. $\bar{y}(s) = \mu(s) \cdot G_u(s) + \bar{d}(s) \cdot G_d(s)$ and which attenuates the disturbance $\bar{d}(s)$, and in a preferred embodiment, could be calculated as a control based on the disturbance observer.

Preferably, the central controller $K(s)$ is defined as:

$$K(s) = \frac{k \cdot F(s) \cdot \tilde{G}_d^{-1}(s)}{1 + k \cdot F(s) \cdot \tilde{G}_d^{-1}(s) \cdot G_u(s)}$$

and the filters $F(s)$ and $F_r(s)$ could be low-pass filters, more specifically, they could be defined as:

$$F(s) = \frac{1}{(\alpha s + 1)^3}$$

$$F_r(s) = \frac{k_r}{(\alpha_r s + 1)^3}$$

wherein $k$, $k_r$, $\alpha$ and $\alpha_r$ are previously calculated parameters and $r(s)$ is a nominal value or target of incremental glucose with respect to $G_b$.

This method further comprises a step of distributing the counter-regulatory action between glucagon administration and rescue carbohydrate administration in the form of:

$$\mu_{cr,v}(s) = (1 - \theta_{cr}) \cdot \mu_{cr}(s)$$

$$\mu_{cr,w}(s) = \theta_{cr} \cdot \mu_{cr}(s)$$

wherein $\theta_{cr}$ is an adjustable parameter between 0 and 1.

In some embodiments, $\theta_{cr}(t)$ may change over time, in some conditions in which one counter-regulatory action is more favourable than the other. For example, it is preferable to administer rescue carbohydrates over glucagon when insulin incorporated is high (such as after a large insulin bolus), or when the accumulated glucagon administration is too large, to avoid side effects such as nausea, or by user preference.

As such, the control actions to be applied are calculated as

$$\bar{u}(s) = \mu_r(s)$$

$$u_T(t) = \bar{u}(t) + u^*(t) + u_b$$

$$v(s) = \frac{G_u(s)}{G_v(s)} \mu_{cr,v}(s)$$

$$w(s) = \frac{G_u(s)}{G_w(s)} \mu_{cr,w}(s)$$

The method of the invention may further comprise a step of implementing a correction to the regulatory and counter-regulatory actions to increase safety when applying the resulting control actions, such that:

$$\mu_r(t) = \bar{u}(t) = \begin{cases} \max(-u_b, \mu_0(t)) & if\ \sigma(t) \geq -\gamma u_b \\ -u_b & if\ \sigma(t) < -\gamma u_b \end{cases}$$

$$\mu_{cr}(t) = \begin{cases} 0\ if\ \sigma(t) \geq -\gamma u_b \\ \min(0, k_{cr} \cdot \mu_0(t) + u_b)\ if\ \sigma(t) < -\gamma u_b \end{cases}$$

wherein $\sigma(t)$ is the function defining the change between regulatory and counter-regulatory actions, as explained above.

Furthermore, the method of the invention may further comprise a step of implementing a correction to $\mu_0$ to prevent glucagon administration as a feedforward action.

In said case, $\mu_{dob}$ is divided into feedback actions ($\mu_{fb}$) and feedforward actions ($\mu_{ff}$) as:

$$\mu_{fb}(s) = K(s) \cdot \big(F_r(s) \cdot r(s) - y(s)\big)$$

$$\mu_{ff}(s) = K(s) \cdot y^*(s)$$

As such, $\mu_0$ is calculated as:

$$\mu_0(t) = \begin{cases} \max\big(-u_b, \mu_{dob}(t)\big) & if\ \mu_{fb}(t) > -u_b \\ \mu_{fb}(t) & if\ \mu_{fb}(t) \leq -u_b \end{cases}$$

As explained above, the control action for rescue carbohydrates could be discretised to administer specific doses of a quantification level ($q_w$). In this case, the discretised control action ($\bar{w}(k)$) could be calculated by means of:

$$\widetilde{w}(k) = \begin{cases} \left\lceil \left| \frac{z_w(k)}{q_w} \right| \right\rceil \cdot q_w\ if\ z_w(k) > \underline{q_w} \wedge HypoFlag \\ 0\ otherwise \end{cases}$$

wherein $\lfloor \cdot \rceil$ indicates the nearest integer operator, $\underline{q_w}$ is a minimum threshold of accumulated carbohydrates to activate a suggestion, $\underline{q_w} < q_w$ and:

$$HypoFlag = \tilde{G}(k) < \tilde{G}(k)_{thr} \ \lor \ G(k) < G(k)_{thr}$$

wherein $\tilde{G}(k)$ is a predicted glucose value in a specific time horizon $t_H$, and $\tilde{G}(k)_{thr}$ and $G(k)_{thr}$ are thresholds for predicted glucose and measured glucose, respectively, which activates the HypoFlag. A prediction for $G(k)$ could be calculated as:

$$\tilde{G}(k) = G(k) + t_H \cdot \delta_G(k)$$

with $\delta_G(k)$ being a discretised filtered glucose derivative, in other words, the discretisation of:

$$\delta_G(s) = \frac{s \cdot G(s)}{\tau_\delta \cdot s + 1}$$

wherein $\tau_\delta > \tau_s$ is a previously determined time constant, and

$$z_w(k) = \hat{w}(k) + \tau_s \cdot BW \cdot w(k)$$

$$\hat{w}(k+1) = \begin{cases} 0 \ if \ z_w(k) > \underline{q_w} \land \neg HypoFlag \\ \left(1 - \frac{\tau_s}{\tau_{cl}}\right) \cdot \left(z_w(k) - \tilde{w}(k)\right) \qquad otherwise \end{cases}$$

wherein $BW$ is the body weight (kg) of the user, $\tau_s > 0$ is a sampling period and $\tau_{cl}$ is a clearance.

Preferably, the threshold $\tilde{G}(k)_{thr}$ is equal to 60 mg/dl and the threshold $G(k)_{thr}$ is equal to 54 mg/dl. Furthermore, the time horizon $t_H$ could be in the range of 5 to 300 minutes, preferably with a value of 60 minutes.

The same methodology could be applied to the control action for administering glucagon as specific doses of a quantification level ($q_v$). In this case, the specific control action ($\tilde{v}(k)$) is calculated by means of:

$$\tilde{v}(k) = \begin{cases} \left\lceil \frac{z_v(k)}{q_v} \right\rceil \cdot q_v \ \ if \ z_v(k) > \underline{q_v} \land HypoFlag \\ 0 \ otherwise \end{cases}$$

wherein $\underline{q_v}$ is a minimum threshold of accumulated glucagon to activate a suggestion, $\underline{q_v} < q_v$ and:

$$HypoFlag = \tilde{G}(k) < \tilde{G}(k)_{thr} \lor G(k) < G(k)_{thr}$$

$$\tilde{G}(k) = G(k) + t_H \cdot \delta_G(k)$$

with $\delta_G(k)$ being a discretised filtered glucose derivative, in other words, the discretisation of:

$$\delta_G(s) = \frac{s \cdot G(s)}{\tau_\delta \cdot s + 1}$$

wherein $\tau_\delta$ is a previously determined time constant and:

$$z_v(k) = \hat{v}(k) + \tau_s \cdot BW \cdot v(k)$$

$$\hat{v}(k+1) = \begin{cases} 0 \; if \; z_v(k) > \underline{q_v} \wedge \neg HypoFlag \\ \left(1 - \dfrac{\tau_s}{\tau_{cl}}\right) \cdot \left(z_v(k) - \tilde{v}(k)\right) \qquad otherwise \end{cases}$$

Preferably, when specific doses are used as control actions, the expected postprandial incremental plasma glucose $y^*(s)$ could be calculated by adding the terms corresponding to said specific doses of counter-regulatory actions, as:

$$y^*(s) = G_u(s) \cdot u^*(s) + G_d(s) \cdot d^*(s) + G_v(s) \cdot \tilde{v}(s) \cdot \eta_v + G_w(s) \cdot \tilde{w}(s) \cdot \eta_w$$

wherein $\eta_v$ and $\eta_w$ are adjustable gains.

Preferably, $\eta_v$ and $\eta_w$ are designed to achieve an increase in the desired maximum output ( $y_{máx}^*$ ) caused when the counter-regulatory action (v, w) is equal to the quantification level ($q_v$, $q_w$), being obtained as:

$$\max\left(\mathcal{L}^{-1}\{q_v \cdot \eta_v \cdot G_v^j(s)\}\right) = y_{max}^*$$

$$\max\left(\mathcal{L}^{-1}\{q_w \cdot \eta_w \cdot G_w^j(s)\}\right) = y_{max}^*$$

wherein $\mathcal{L}^{-1}\{\cdot\}$ represents an inverse Laplace transform operator and $y_{máx}^*$ is the desired output variation due to the quantification level ($q_v$, $q_w$).

Preferably, the expected postprandial incremental plasma glucose could become saturated $y_{sat}^*(t)$, resulting as:

$$y_{sat}^*(t) = \begin{cases} y_{high}^* - G_b \quad if \; y^*(t) \ge y_{high}^* - G_b \\ y^*(t) \; if \; y_{high}^* - G_b > y^*(t) > y_{low}^* - G_b \\ y_{low}^* - G_b \quad if \; y^*(t) \le y_{low}^* - G_b \end{cases}$$

wherein $y_{high}^*$ and $y_{low}^*$ and previously determined upper and lower thresholds.

Optionally, the adjustable gain k, mentioned above, is defined as $k = 0{,}65 \times 6 \times (1/\,CR^j)$.

As an alternative, the adjustable gain k is defined as:

$$k = \min_{t>0} \frac{\mathcal{L}^{-1}\{G_d(s)\}(t) - \dfrac{y^{min}}{\tilde{M}}}{\mathcal{L}^{-1}\{F(s) \cdot G_u(s)\}(t - l_u)}$$

wherein $\mathcal{L}^{-1}$ is the inverse Laplace transform, and $y^{min}$ and $\tilde{M} > 0$ are adjustable parameters, with $\tilde{M}$ representing the expected meal size in the worst case, and $y^{min}$ the tolerable lower limit for the incremental postprandial glucose response to that meal $\tilde{M}$.

Optionally, $\tilde{M}$ is defined as the size of the meal notified by the patient ($M^*$) whenever an optional meal notification is made, and $y^{min}$ is defined as the lower tolerable limit for the incremental postprandial glucose response to that meal $M^*$, maintaining the calculated value of $k$ for an adjustable time window from the time the meal was notified.

In some embodiments, the insulin bolus administered ($u^*$) is calculated from the meal size notified by the patient ($M^*$) as a bolus defined by $u^*(s) = C(s)M^*(s)$, wherein $C(s)$ is a feedforward controller that could be defined as:

$$C(s) = \frac{k_d}{k_u}$$

As an alternative, the feedforward controller C(s) could follow a superbolus strategy defined as:

$$C(s) = \frac{k_d}{k_u} + u_b \cdot T_\sigma - \frac{u_b}{s}(1 - e^{-T_\sigma s})$$

with $T_\sigma > 0$ being a time period in which the pump is turned off.

The invention also relates to an artificial pancreas system incorporating the method for controlling blood glucose as defined above, comprising:

- a pump, to deliver an automated insulin infusion, according to coordinated control action ($u_T$), and/or an automated glucagon infusion, according to coordinated control action ($v$, $\tilde{v}$);
- a continuous glucose monitor to detect the plasma glucose signal (G(t)); and
- a first computing unit configured to perform the steps of the method of the invention.

As an alternative, the system may comprise a second pump to deliver the automated glucagon infusion.

In alternative embodiments, the artificial pancreas system comprises a display to notify recommended control actions for the administration of glucagon and/or rescue carbohydrates. In these cases, the system may also comprise a pen device for the patient to deliver the recommended dose of glucagon.

The invention also relates to a computer program adapted to carry out the steps of the defined method using the computing unit of the artificial pancreas system, and to a computer-readable storage medium comprising said computer program.

## DESCRIPTION OF THE DRAWINGS

To complement the description being made and to help to better understand the features of the invention according to a preferred practical embodiment thereof, a set of drawings is attached as an integral part of said description in which the following has been depicted with an illustrative and non-limiting character:

Figure 1 shows a schematic depiction of the control strategy followed by the method of the invention.
Figure 2 shows a graph of the percentage of time in which glucose is in a specific range.
Figure 3 shows the control actions calculated for the configurations of Figure 2 according to the method of the invention.

## PREFERRED EMBODIMENTS OF THE INVENTION

To obtain parameters, patient-oriented control models based on an identification method are presented, resulting in personalised gains based on readily available relevant clinical parameters of the patient.

Interaction between glucose and insulin is a complex system, which can be represented by means of a high-dimensional non-linear model. However, for control design purposes, simple models with few personalised parameters are often used.

The proposed AP is validated with the cohort of 10 adults from the academic version of the UVa/Padova simulator in a challenging scenario with meals and exercise events.

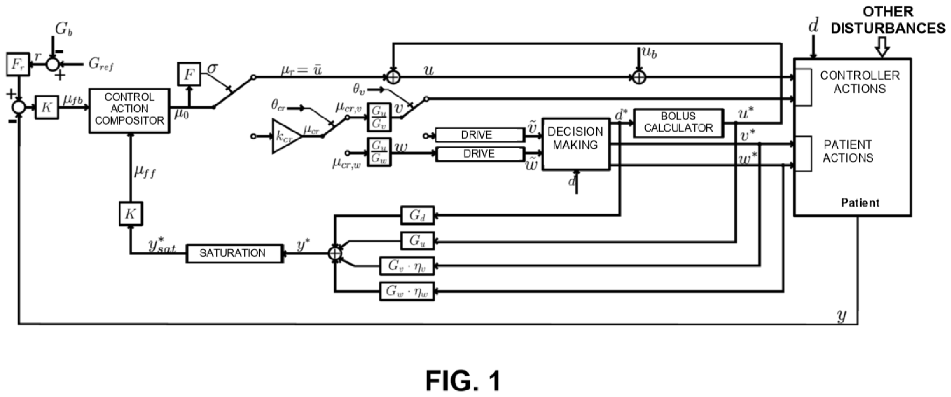

Figure 1 depicts the proposed fully autonomous coordinated artificial pancreas system. The core of the system comprises four basic components: asymmetric coordinated control (A), the carbohydrate and glucagon recommendation module (B), the patient action adaptation module (C), and the central controller and pre-filter (D). Asymmetric coordinated control distributes the output of the patient action adaptation module into an insulin infusion, $u(t)$, and a counter-regulatory control action that decreases and increases glucose levels, respectively. As a counter-regulatory control action, the user can configure the controller to provide an automatic glucagon infusion ($v(t)$), a suggestion for a quantified dose of glucagon ($\tilde{v}(t)$) to be administered, for example, with a pen, or a suggestion for carbohydrate intake ($\bar{w}(t)$), as derived from the carbohydrate and glucagon recommendation module. The central controller is designed for operation without meal notifications, but the system is able to also manage notified meals through the patient action adaptation module. This module also adapts the output of the central controller when the user manually administers a quantified counter-regulatory action, such as carbohydrate intake or glucagon injection.

The system design assumes the following simplified relationship between incremental glucose $y$ (mg/dl) with respect to the baseline state, the incremental insulin infusion rate u (pmol/kg/min) with respect to baseline infusion $u_b$, the glucagon infusion rate v (mg/kg/min), and the rescue carbohydrate administration rate w (mg/kg/min):

$$y(s) = G_u(s) \cdot u(s) + G_v(s) \cdot v(s) + G_w(s) \cdot w(s) + G_d(s) \cdot d(s) \quad (1)$$

where $d(s)$ corresponds to the oral carbohydrate intake (mg/kg/min) with size M modelled with a pulse signal, in other words, $d(s) = M$. Linear transfer functions with time delay $G_d(s)$, $G_u(s)$, $G_v(s)$, and $G_w(s)$ are described by means of

$$G_i(s) = \tilde{G}_i(s) \cdot e^{-15s}, \; i \in \{d, u, v, w\} \; (2)$$

$$\tilde{G}_i(s) = \frac{k_i^j}{(\tau_{1i}s + 1)^2(\tau_{2i}s + 1)}$$

where the superscript $j$ indicates a gain adapted to the patient. Control inputs are subjected to saturation restrictions $u(t) \geq -u_b$ and $v(t)$, $w(t) \geq 0$ for all $t$. The dynamics in (2) assume three approximations to simplify the identification of the models:

1) two compartments represent the absorption of the input ($u$, $d$, $v$, or $w$),
2) one compartment simulates the effect of this input on glucose, and
3) all the models have an identical delay equal to 15 minutes.

It should be noted that personalised models of similar gain are conventional for diabetes control purposes.
The above model was identified for the 10 virtual adults in the UVa/Padova distribution version. The parameters of the control-oriented patient model corresponding to the virtual adults identified in the UVa/Padova simulator are shown in Table I.

Table I.

| Subject | $k_d^j$ | $k_u^j$ | $k_v^j$ | $k_w^j$ |
|---|---|---|---|---|
| $j$ | $\left(\dfrac{\text{mg/dl}}{\text{mg/kg/min}}\right)$ | $\left(\dfrac{\text{mg/dl}}{\text{pmol/kg/min}}\right)$ | $\left(\dfrac{\text{mg/dl}}{\text{mg/kg/min}}\right)$ | $\left(\dfrac{\text{mg/dl}}{\text{mg/kg/min}}\right)$ |
| 1 | $5.88 \cdot 10^4$ | -187.62 | $7.59 \cdot 10^6$ | $9.85 \cdot 10^4$ |
| 2 | $4.92 \cdot 10^4$ | -184.43 | $1.49 \cdot 10^7$ | $8.71 \cdot 10^4$ |
| 3 | $7.46 \cdot 10^4$ | -180.94 | $5.22 \cdot 10^7$ | $8.21 \cdot 10^4$ |
| 4 | $6.62 \cdot 10^4$ | -217.40 | $4.48 \cdot 10^6$ | $6.85 \cdot 10^4$ |
| 5 | $9.28 \cdot 10^4$ | -208.32 | $7.80 \cdot 10^6$ | $7.30 \cdot 10^4$ |
| 6 | $1.09 \cdot 10^5$ | -163.31 | $3.26 \cdot 10^6$ | $9.05 \cdot 10^4$ |
| 7 | $7.75 \cdot 10^4$ | -234.43 | $1.97 \cdot 10^6$ | $7.39 \cdot 10^4$ |
| 8 | $1.13 \cdot 10^5$ | -165.93 | $3.65 \cdot 10^6$ | $1.17 \cdot 10^5$ |
| 9 | $6.60 \cdot 10^4$ | -217.41 | $1.32 \cdot 10^7$ | $6.22 \cdot 10^4$ |
| 10 | $7.84 \cdot 10^4$ | -180.06 | $9.46 \cdot 10^6$ | $8.57 \cdot 10^4$ |

| $\tau_{1d}$ (min) | $\tau_{1u}$ (min) | $\tau_{1v}$ (min) | $\tau_{1w}$ (min) | $\tau_{2d}$ (min) | $\tau_{2u}$ (min) | $\tau_{2v}$ (min) | $\tau_{2w}$ (min) |
|---|---|---|---|---|---|---|---|
| 25 | 55 | 27.5 | 12.5 | 380 | 380 | 235 | 315 |

The control strategy presented should allow patients to provide a meal notification, resulting in the administration of an insulin bolus, if desired. However, doing so without notifying the feedback controller may result in an insulin overdose. To avoid this situation, the central controller is notified about the meal through a non-interactive feedback-feedforward scheme implemented in the patient action adaptation module. The variance of the expected output is defined as

$$y^*(s) = G_d(s) \cdot d^*(s) + G_u(s) \cdot u^*(s) \; (3)$$

based on an insulin bolus u*(s) = B* calculated by

$$u^*(s) = v \frac{d^*(s)}{CIR} \frac{1}{6000BW}$$

where $d^*(s) = M^*$ is the carbohydrate content of the notified meal (g), $CIR$ is the ratio of carbohydrates to insulin (g/U), $BW$ is the body weight (kg), $\upsilon \in (0, 1)$ is an attenuation factor and the term $(6000BW)^{-1}$ converts insulin units from U to pmol/kg. Subtracting (1) and (3) results in

$$\bar{y}(s) \;=\; G_u(s) \cdot \bar{u}(s) + G_v(s) \cdot v(s) + G_w(s) \cdot w(s) + G_d(s) \cdot \bar{d}(s) \quad (4)$$

where $\bar{y}(s) = y(s) - y^*(s)$, $\bar{u}(s) = u(s) - u^*(s)$, and $\bar{d}(s) = d(s) - d^*(s)$. The controller can now be designed based on model (4), which already incorporates the feedforward actions performed by the patient. Note that, in the ideal case where the patient provides notification about the exact meal size, resulting in the administration of an insulin bolus of a suitable size, the feedback controller shall have no effect as long as the output follows the expected path, avoiding, therefore, unwanted interactions between feedback and feedforward actions.

Having more manipulated inputs than regulated outputs, as is the case with the MISO (multiple input single output) model (4), can be exploited to improve performance under different operating conditions and manage input saturation, but also poses difficulties in the design procedure. In order to simplify this task and considering the nature of the different control actions, the model (4) is conveniently rewritten as

$$\bar{y}(s) \;=\; G_u(s) \cdot \mu(s) + G_d(s) \cdot \bar{d}(s) \quad (5)$$

where

$$\mu(s) = \bar{u}(s) + \frac{G_v(s)}{G_u(s)} v(s) + \frac{G_w(s)}{G_u(s)} w(s)$$

is considered a virtual control action comprising both regulatory and counter-regulatory actions, defined by

$$\mu(s) \triangleq \mu_r(s) + \mu_{cr}(s) \quad (6)$$

$$\mu_r(s) \triangleq \bar{u}(s) \quad (7)$$

$$\mu_{cr}(s) \triangleq \mu_{cr,v}(s) + \mu_{cr,w}(s) \quad (8)$$

including the last two terms, representing the counter-regulatory effect implemented by means of glucagon infusion (v) or carbohydrate intake (w), provided by

$$\mu_{cr,v}(s) \triangleq \frac{G_v(s)}{G_u(s)} v(s)$$

$$\mu_{cr,w}(s) \triangleq \frac{G_w(s)}{G_u(s)} w(s)$$

The counter-regulatory mode should be more aggressive to compensate for any drop in glucose levels that could have serious consequences for the patient. Let $\mu_0(s)$ be the result of the patient action adaptation module. The final control effort to distribute, $\mu$, is defined as

$$\mu(t) \triangleq \begin{cases} \mu_0(t) \; if \; \sigma(t) \geq -\gamma u_b \\ k_{cr} \cdot \mu_0(t) \; if \; \sigma(t) < -\gamma u_b \end{cases} \quad (9)$$

where $\mu_0$ will be defined below. The following coordinated scheme is proposed

$$\mu_r(t) = \begin{cases} \max(-u_b, \mu_0(t)) & if \; \sigma(t) \geq -\gamma u_b \\ -u_b & if \; \sigma(t) < -\gamma u_b \end{cases} \quad (10)$$

$$\mu_{cr}(t) = \begin{cases} 0 \ if \ \sigma(t) \geq -\gamma u_b \\ \min(0, k_{cr} \cdot \mu_0(t) + u_b) \ if \ \sigma(t) < -\gamma u_b \end{cases} \quad (11)$$

with a controller gain increased by $k_{cr} \geq 1$ in the counter-regulatory mode. Note that this definition complies with the restriction

$$\mu(t) = \mu_r(t) + \mu_{cr}(t), \ \text{for all t}$$

imposed by equation (6), unless saturations are at work. Furthermore, to comply with (8) and, therefore, ensure coordination of the two available counter-regulatory actions, the following distribution is selected:

$$\mu_{cr,v}(s) = (1 - \theta_{cr}) \cdot \mu_{cr}(s), \ \mu_{cr,w}(s) = \theta_{cr} \cdot \mu_{cr}(s) \quad (12)$$

where $\theta_{cr} \in \{0, 1\}$ can be adjusted, in a time-varying manner, to choose between glucagon or rescue carbohydrates.

The above coordination logic incorporates two mechanisms to minimise the consumption of counter-regulatory actions. On the one hand, switching is performed on a filtered signal $\sigma$. This signal is calculated by applying a moving average filter with a three-sample window to the signal. $\mu_0$. This is to avoid unwanted activation of the counter-regulatory mode due to the noise contained in the control action. Moreover, the threshold for activating the counter-regulatory mode is shifted slightly by means of an adjustable factor $\gamma$. This modification implies the existence of a small dead zone $-y \cdot u_b < \sigma(s) < -u_b$ in which no counter-regulatory actions are executed. The *min* and max functions are used only to ensure the feasibility of the resulting control actions. Lastly, with $\mu_r(s)$, $\mu_{cr,v}(s)$, and $\mu_{cr,w}(s)$ as defined above, the control signals ensuring closed-loop coordination can be calculated from (13) - (15) as

$$\bar{u}(s) = \mu_r(s), \ v(s) = \frac{G_u(s)}{G_v(s)}\mu_{cr,v}(s), \ w(s) = \frac{G_u(s)}{G_w(s)}\mu_{cr,w}(s).$$

A positive insulin pulse, in other words, an insulin bolus is the optimal insulin infusion to limit the postprandial peak while minimising at the same time glucose insufficiency. From the equivalent model (5) the following central controller can be derived:

$$\mu_{dob}(s) = K(s) \cdot \left(F_r(s) \cdot r(s) - y(s)\right) + K(s) \cdot y^*(s) \quad (16)$$

where

$$K(s) = \frac{k \cdot F(s)\tilde{G}_d^{-1}(s)}{1 + k \cdot F(s)\tilde{G}_d^{-1}(s)G_u(s)}$$

$$F(s) = \frac{1}{(\alpha s + 1)^3}$$

$$F_r(s) = \frac{k_r}{(\alpha_r s + 1)^3}$$

are strictly suitable filters with adjustable parameters $k, k_r, \alpha, \alpha_r > 0$. The control action (16) can be rewritten as:

$$\mu_{dob}(s) = \mu_{fb}(s) + \mu_{ff}(s)$$

$$\mu_{fb}(s) = K(s) \cdot \left(F_r(s) \cdot r(s) - y(s)\right)$$

$$\mu_{ff}(s) = K(s) \cdot y^*(s) \quad (17)$$

which shows that the output of the central controller is the contribution of two terms: one resulting from the tracking error $\mu_{fb}$, and the other being a correction term due to the feedforward actions provided $\mu_{ff}$. Note that, in some cases, for example, during a postprandial period after a pre-meal bolus, an overestimated $y^*$ may lead to counter-regulatory control action, which is not desirable. To avoid this, both contributions are combined as follows:

$$\mu_0(t) = \begin{cases} \max\left(-u_b, \mu_{dob}(t)\right) & if\ \mu_{fb}(t) > -u_b \\ \mu_{fb}(t) & if\ \mu_{fb}(t) \leq -u_b \end{cases} \quad (18)$$

Rescue carbohydrate suggestions must be provided as specific quantified doses rather than at a continuous rate. Therefore, the continuous signal w must be converted into quantified pulses. Let $\tau_s > 0$ be the sampling period, then the recommended carbohydrate intake at the time of sampling k, indicated as $\tilde{w}(k)$, is given by

$$\tilde{w}(k) = \begin{cases} \left\lfloor \left| \frac{z_w(k)}{q_w} \right| \right\rfloor \cdot q_w\ if\ \left(z_w(k) > \underline{q}_w\right) \wedge HypoFlag \\ 0\ otherwise \end{cases} \quad (19)$$

where $\lfloor \cdot \rfloor$ indicates the nearest integer operator, $q_w$ Is the quantification level and $\underline{q}_w < q_w$ is the minimum threshold of accumulated carbohydrates to activate a suggestion. The signal $z_w(k)$ is an auxiliary variable defined by

$$z_w(k) = \hat{w}(k) + \tau_s \cdot BW \cdot w(k) \quad (20)$$

wherein $\tau_\delta$ is a previously determined time constant that depends on the rate of accumulated carbohydrates with clearance $\tau_{cl} > \tau_s$ described by

$$\hat{w}(k+1) = \begin{cases} 0\ if\ \left(z_w(k) > \underline{q}_w\right) \wedge \neg HypoFlag \\ \left(1 - \frac{\tau_s}{\tau_{cl}}\right) \cdot \left(z_w(k) - \tilde{w}(k)\right) & otherwise \end{cases}$$

*HypoFlag* is an alert that indicates the risk of hypoglycaemia. It corresponds to the condition

$$HypoFlag = \left(\tilde{G}(k) < 60\right)\ \vee\ \left(G(k) < 54\right) \quad (21)$$

where $G(k)$ is the actual reading of the continuous glucose monitor (CGM) and $\tilde{G}(k)$ is the 1 h anticipated glucose prediction calculated with

$$\tilde{G}(k) = G(k) + 60 \cdot \delta_G(k) \quad (22)$$

where $\delta_G(k)$ is a discretisation of $\frac{s \cdot G(s)}{\tau_\delta \cdot s + 1}$, in other words, the glucose derivative filtered with a time constant low pass filter $\tau_\delta$.

The above quantification scheme will activate a carbohydrate recommendation, multiple of $q_w$ mg, as long as $z_w(k)$ reaches $\underline{q}_w$. In turn, the suggested carbohydrates will be subtracted from the cumulative signal $\tilde{w}(k)$ to compensate for the excess. To reduce the delay of the accumulation process, $\overline{q}_w$ is set below $q_w$. A consequence of quantification delay is that the controller may activate a suggestion too late when the continuous equivalent w(k) would be close to 0. The condition *HypoFlag* in (19) is established to reduce the risk of these late suggestions. Furthermore, $\hat{w}(k)$ includes a forgetting factor and is set to zero when it is large enough to activate a suggestion, but the condition *HypoFlag* is not met.

Unlike rescue carbohydrate intake, the glucagon infusion rate can be administered automatically through a pump (a pump other than an insulin pump or a dual-chamber pump). The system can administer glucagon continuously and at the same time have quantified carbohydrate suggestions. However, some patients may prefer manual injections of low-dose glucagon as a non-caloric alternative to recover from mild hypoglycaemia without needing an additional (or more complex) pump. The glucagon quantification strategy implements the equivalent equations of the carbohydrate quantification scheme by replacing in a corresponding manner $z_w$, $\tilde{w}$, $\hat{w}$, $q_w$, and $\underline{q}_w$ with $z_v$, $\tilde{v}$, $\hat{v}$, $q_v$, and $\underline{q}_v$.

The quantified counter-regulatory action may differ from that calculated by the central controller. To inform the central controller of this situation, the non-interactive feedback-feedforward scheme is modified by redefining the expected output variation in (3) as follows:

$$y^*(s) = G_d(s) \cdot d^*(s) + G_u(s) \cdot u^*(s) + G_v(s) \cdot \tilde{v}(s) \cdot \eta_v + G_w(s) \cdot \tilde{w}(s) \cdot \eta_w \quad (23)$$

where $\eta_v$ and $\eta_w$ define the increase in the desired maximum output caused when the counter-regulatory action is equivalent to the corresponding quantification level. Hence, these parameters can be derived from

$$\max\left(\mathcal{L}^{-1}\{q_v \cdot \eta_v \cdot G_v^j(s)\}\right) = y_{max}^*$$

$$\max\left(\mathcal{L}^{-1}\{q_w \cdot \eta_w \cdot G_w^j(s)\}\right) = y_{max}^*$$

where $\mathcal{L}^{-1}\{\cdot\}$ indicates the inverse Laplace transform operator and $y_{máx}^*$ indicates the desired output variation due to $q_v$ or $q_w$. Furthermore, to prevent the output from accidentally causing hyperglycaemia or hypoglycaemia, the signal y* is saturated as follows:

$$y_{sat}^*(t) = \begin{cases} 180 - G_b & if \ y^*(t) \geq 180 - G_b \\ y^*(t) & if \ 180 - G_b > y^*(t) > 70 - G_b \ (24) \\ 70 - G_b & if \ y^*(t) \leq 70 - G_b \end{cases}$$

It should be noted that the expected output variation modified in (24) is a practical consideration for implementation purposes; the central controller design is still based on the factored model (5) derived from (4).

Table II.

| Subject $j$ | $k^j$ $\left(\dfrac{\text{mg/dl}}{\text{pmol/kg/min}}\right)$ | $k^j_{cr}$ (dimensionless) | $\gamma^j$ (dimensionless) | $\alpha$ (min) | $k_r$ $\left(\dfrac{\text{mg/dl}}{\text{mg/dl}}\right)$ |
|---|---|---|---|---|---|
| 1 | $2.192 \cdot 10^2$ | | | | |
| 2 | $1.868 \cdot 10^2$ | | | | |
| 3 | $2.886 \cdot 10^2$ | | | | |
| 4 | $2.132 \cdot 10^2$ | | | | |
| 5 | $3.118 \cdot 10^2$ | | | | |
| 6 | $4.669 \cdot 10^2$ | 3.895 | 1.335 | 8 | 1 |
| 7 | $2.315 \cdot 10^2$ | | | | |
| 8 | $4.777 \cdot 10^2$ | | | | |
| 9 | $2.125 \cdot 10^2$ | | | | |
| 10 | $3.050 \cdot 10^2$ | | | | |
| $\alpha_r, \tau_{cl}, \tau_\delta$ (min) | $q_v$ (mg) | $\underline{q}_v$ (mg) | $q_w$ (mg) | $\underline{q}_w$ (mg) | $v$ (dimensionless) |
| 30 | 0.08 | 0.026 | 15000 | 7500 | 0.8 |

Table II includes the controller parameters. The gain $k$ of the central controller was individualised by subject $j$ according to the following formula:

$$k^j = 6000 \frac{\eta}{CIR} \quad (25)$$

where $\eta$ is a safety factor established at 0.7. The values of $\alpha, \alpha_r$ and $v$ in Table II were derived through simulations. Since the counter-regulation gain is adjusted $k_{cr}$ and the factor $\gamma$ were heuristic, initially, an optimisation-based strategy was used to individualise these parameters. However, optimisation-based adjustments are not feasible for clinical trials; therefore, a population value was also assessed by taking the median of the optimal values for each parameter. No relevant differences were found in the simulations between the use of the optimal adjustment and the population value. Hence, population parameters were selected for this work for reasons of simplicity. As for quantification-related parameters, the carbohydrate quantification level was set at the conventional dose in commercial gels, in other words, 15 g. Glucagon suggestions were quantified at 0.08 mg, since this quantification level resulted in safe and effective control in a recent clinical trial. The remaining quantification-related parameters in Table II - $q_v$, $q_w$, and $\tau_{cl}$ -, and the thresholds that define the condition *HypoFlag,* were adjusted by means of simulations. It can be noted based on Table II that $q_v$ and $q_w$ were both established at one-third of the corresponding quantification level.

As for the results obtained, six configurations of the proposed controller - hybrid system with glucagon, hybrid system with carbohydrate suggestions, the system without meal notification with glucagon, and the system without meal notification with carbohydrate suggestions, where glucagon was considered to be administered as a suggestion or continuous infusion - they were simulated for the 10 virtual adults included in an extended version of the UVa/Padova simulator.

The scenario included 14 days with 3 meals per day, randomised in terms of carbohydrate content and time: 37.0 [30.0, 45.0] g (median [percentile 25, percentile 75]) at 8:05 [7:45, 8:15] h, 53.0 [36.0, 58.0] g at 13:05 [12:50, 13:15] h, and 75.5 [53.0, 90.0] g at 19:50 [19:35, 20:10] h. In addition, 8 aerobic exercise sessions were randomly scheduled at 19:17 [14:40, 22:10] h on alternate days, from day one, with a duration of 55.0 [50.0, 60.0] minutes and an intensity of 48.5 [47.5, 53.0]% of maximum oxygen volume. The effect of exercise on glucose was implemented by increasing insulin sensitivity. Lastly, the controller configurations were tested for noise (built-in sensor model *dexcom25*) and the following sources of variability that were implemented in the academic version of the simulator: the nominal values of the meal absorption rate and carbohydrate bioavailability parameters of the meal absorption model varied per meal with a uniform distribution of $\pm 30\%$ and $\pm 10\%$ respectively; the nominal values of the parameters describing insulin pharmacokinetics were modified according to a uniform distribution of $\pm 30\%$ for each meal; circadian variation in insulin sensitivity was represented with a sinusoidal change in a 24 h period with random amplitude following a uniform $\pm 30\%$ and random phase; and an erroneous estimation of the carbohydrate content of the meal was implemented. It should be noted that, for controller configuration with carbohydrate and glucagon suggestions, the user follows the recommendation (i.e., take carbohydrates or administer glucagon) simultaneously with the suggestion. Even more ideal, this assumption is considered in other works in the literature. The performance of the controller configurations was evaluated through the % of time in the range, below the range, or above the range, and daily consumption of insulin, glucagon, and carbohydrates.

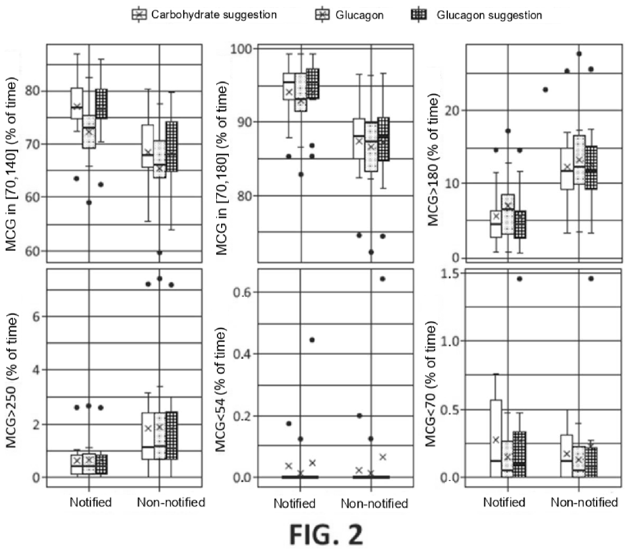

Figure 2 shows a graph of the percentage of time in which glucose is in a specific range. The lower and upper boxes represent the percentiles 25 and 75, respectively, and the horizontal thick black lines correspond to the median. The crosses represent means.

The ends of the upper and lower whiskers represent the largest and smallest values, respectively, not more than (at most) 1.5 times the interquartile range. Black dots beyond the whisker correspond to outliers.

Notified and non-notified meals are considered. Furthermore, glucagon continuously administered by means of a pump (glucagon) and quantified glucagon suggestions by means of a pen (glucagon suggestion) are assessed. Carbohydrates are always quantified and suggested to the patient for manual intake (carbohydrate suggestion).

All controller configurations are able to manage both non-notified meals and exercise events, as shown in Figure 2, with mean values within their respective ranges. Episodes of hypoglycaemia are rare in all configurations. Only subjects 3 and 6 had episodes of CGM <54 mg/dl, although their time in said range was below 0.70%. Six subjects had episodes with CGM <70, but were clinically acceptable. The patient with the longest time in hypoglycaemia (subject 3) was only 1.6% below 70 mg/dl. The reduction of time in hypoglycaemia is achieved without excessive application of counter-regulatory control actions.

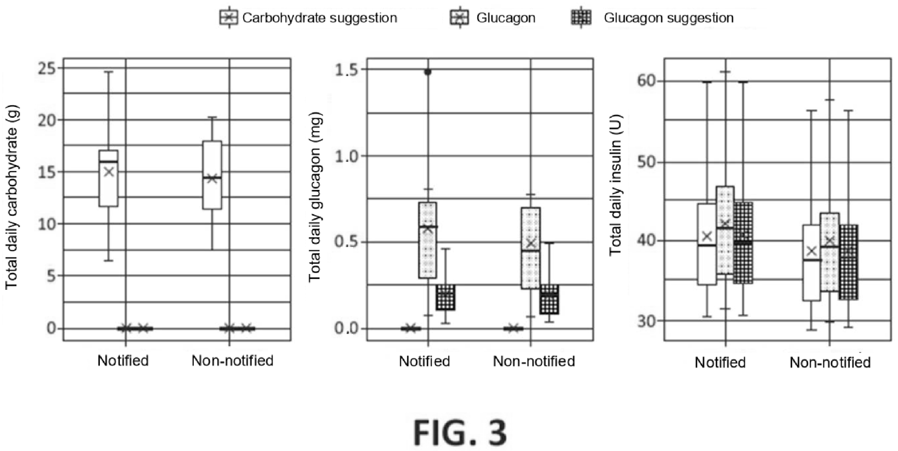

Figure 3 shows the control actions calculated for the configurations of Figure 2 according to the method of the invention.

In the glucagon configuration, the median daily dose required is less than 1 mg, as shown in Figure 3, a threshold where subjects often experience nausea. The daily dose required in the carbohydrate configuration is similar to that of other controllers in the literature evaluated in equivalent synthetic scenarios.

## Claims

1. A method for controlling glucose in a flexibly structured dual-hormone artificial pancreas which is able to manage optional meal notifications and optional exercise notifications by means of a coordinated control action, the method comprising the steps of:

    - measuring a plasma glucose signal ($G(t)$) by means of a continuous glucose monitor (CGM);
    - calculating an incremental plasma glucose measurement by means of:

$$y(t) = \ G(t) - \ G_b$$

wherein $G_b$ is a baseline glucose value;

- defining a model for incremental plasma glucose (y), taking into account a carbohydrate intake (d), an incremental insulin infusion (u) with respect to a baseline infusion $u_b$, a glucagon administration (v), and a rescue carbohydrate administration (w), as:

$$y(s) = G_u(s) \cdot u(s) + G_v(s) \cdot v(s) + G_w(s) \cdot w(s) + G_d(s) \cdot d(s)$$

wherein $u(t) \geq -u_b$, $v(t) \geq 0$ and $w(t) \geq 0$; and wherein $G_u$, $G_v$, $G_w$, $G_d$ are time-invariant linear transfer functions, correlating carbohydrate intake (d), incremental insulin infusion (u), glucagon administration (v), and rescue carbohydrate administration (w) with incremental plasma glucose (y), and are subject to the restriction that the order of the numerator of $G_u(s)/G_y(s)$ and $G_u(s)/G_w(s)$ does not exceed the order of the denominator;

- defining a carbohydrate intake as:

$$d^*(s) = \begin{cases} M^* & if\ meal\ is\ notified \\ 0 & if\ meal\ is\ not\ notified \end{cases}$$

where $M^*$ is an estimated carbohydrate content notified by a patient;

- defining an expected postprandial incremental plasma glucose $y^*(s)$, as:

$$y^*(s) = G_u(s) \cdot u^*(s) + G_d(s) \cdot d^*(s)$$

wherein $u^*(s)$ is the insulin bolus administered in association with the patient's manual meal notification;

- defining a corrected incremental plasma glucose $\bar{y}(s)$, a corrected insulin infusión $\bar{u}(s)$, and a corrected carbohydrate intake $\bar{d}(s)$ as:

$$\bar{y}(s) = \ y(s) - y^*(s)$$

$$\bar{u}(s) = \ u(s) - u^*(s)$$

$$\bar{d}(s) = \ d(s) - d^*(s)$$

$$\bar{y}(s) = G_u(s) \cdot \bar{u}(s) + G_v(s) \cdot v(s) + G_w(s) \cdot w(s) + G_d(s) \cdot \bar{d}(s)$$

- defining a virtual control action $\mu(s)$ as:

$$\mu(s) = \bar{u}(s) + \frac{G_v(s)}{G_u(s)} v(s) + \frac{G_w(s)}{G_u(s)} w(s)$$

such that:

$$\bar{y}(s) = G_u(s) \cdot \mu(s) + G_d(s) \cdot \bar{d}(s)$$

- dividing the control action $\mu(s)$ into regulatory control actions $\mu_r$ and counter-regulatory control actions $\mu_{cr}$:

$$\mu(s) \triangleq \mu_r(s) + \mu_{cr}(s)$$

- distributing the counter-regulatory action as

$$\mu_{cr}(s) \triangleq \mu_{cr,v}(s) + \mu_{cr,w}(s)$$

wherein $\mu_{cr,v}$ represents the counter-regulatory effect implemented by means of glucagon infusion and $\mu_{cr,w}$ represents the counter-regulatory effect implemented by means of carbohydrate intake, such that:

$$\mu_{cr,v}(s) \triangleq \frac{G_v(s)}{G_u(s)} v(s)$$

$$\mu_{cr,w}(s) \triangleq \frac{G_w(s)}{G_u(s)} w(s)$$

- calculating regulatory and counter-regulatory actions as:

$$\mu_r(t) = \begin{cases} \mu_0(t) \; if \; \sigma(t) \geq -\gamma u_b \\ -u_b \qquad\quad if \; \sigma(t) < -\gamma u_b \end{cases}$$

$$\mu_{cr}(t) = \begin{cases} 0 \; if \; \sigma(t) \geq -\gamma u_b \\ k_{cr} \cdot \mu_0(t) + u_b \; \; if \; \sigma(t) < -\gamma u_b \end{cases}$$

wherein $\sigma(t)$ is a function defining a change between regulatory and counter-regulatory actions, and $\gamma$ is an adjustable factor, and wherein $k_{cr}$ is a previously obtained controller gain, and $\mu_0$ is defined as:

$$\mu_0(s) = \mu_{dob}(s)$$

with $\mu_{dob}$ being calculated as a 2-DOF feedback controller with a nominal value pre-filter $F_r(s)$ as:

$$\mu_{dob}(s) = K(s) \cdot \big(F_r(s) \cdot r(s) - \bar{y}(s)\big)$$

$$\mu_{dob}(s) = K(s) \cdot \big(F_r(s) \cdot r(s) - y(s)\big) + K(s) \cdot y^*(s)$$

and wherein $K(s)$ is a central linear controller designed to stabilise the system $\bar{y}(s) = \mu(s) \cdot G_u(s) + \bar{d}(s) \cdot G_d(s)$ and attenuating the disturbance $\bar{d}(s)$,

- calculating the counter-regulatory actions in the form of:

$$\mu_{cr,v}(s) = (1 - \theta_{cr}) \cdot \mu_{cr}(s)$$

$$\mu_{cr,w}(s) = \theta_{cr} \cdot \mu_{cr}(s)$$

wherein $\theta_{cr} \in [0,1]$ is an adjustable parameter; y
- calculating the control actions as:

$$\bar{u}(s) = \mu_r(s)$$

$$u(t) = \bar{u}(t) + u^*(t) + u_b$$

$$v(s) = \frac{G_u(s)}{G_v(s)} \mu_{cr,v}(s)$$

$$w(s) = \frac{G_u(s)}{G_w(s)} \mu_{cr,w}(s)$$

2. The method according to claim 1, wherein the models $G_u$, $G_v$, $G_w$, $G_d$ are obtained as:

$$G_u(s) = \tilde{G}_u(s) \cdot e^{-l_u s}$$

$$G_v(s) = \tilde{G}_v(s) \cdot e^{-l_v s}$$

$$G_w(s) = \tilde{G}_w(s) \cdot e^{-l_w s}$$

$$G_d(s) = \tilde{G}_d(s) \cdot e^{-l_d s}$$

where $\tilde{G}_u(s)$, $\tilde{G}_v(s)$, $\tilde{G}_w(s)$ and $\tilde{G}_d(s)$ are transfer functions in the form:

$$\tilde{G}_u(s) = \frac{k_j^u}{(\tau_{1u} + 1)^2 (\tau_{2u} s + 1)}$$

$$\tilde{G}_v(s) = \frac{k_j^v}{(\tau_{1v} + 1)^2 (\tau_{2v} s + 1)}$$

$$\tilde{G}_w(s) = \frac{k_j^w}{(\tau_{1w} + 1)^2 (\tau_{2w} s + 1)}$$

$$\tilde{G}_d(s) = \frac{k_j^d}{(\tau_{1d} + 1)^2 (\tau_{2d} s + 1)}$$

wherein $l_u$, $l_v$, $l_w$ and $l_u$ are parameters representing delays, $k_j^u$, $k_j^v$, $k_j^w$, $k_j^d$, $\tau_{1u}$, $\tau_{2u}$, $\tau_{1v}$, $\tau_{2v}$, $\tau_{1w}$, $\tau_{2w}$, $\tau_{1d}$ and $\tau_{2d}$ are previously obtained parameters and j is an index representing a patient.

3. The method according to any of claims 1 to 2, further comprising a step of implementing a correction to regulatory and counter-regulatory actions in the form of:

$$\mu_r(t) = \begin{cases} \max(-u_b, \mu_0(t)) & if \ \sigma(t) \geq -\gamma u_b \\ -u_b & if \ \sigma(t) < -\gamma u_b \end{cases}$$

$$\mu_{cr}(t) = \begin{cases} 0 \ if \ \sigma(t) \geq -\gamma u_b \\ \min(0, k_{cr} \cdot \mu_0(t) + u_b) \ if \ \sigma(t) < -\gamma u_b \end{cases}$$

wherein $\sigma$ is a function defining the change between regulatory and counter-regulatory actions and $\gamma$ is an adjustable factor.

4. The method according to any of claims 1 to 3, wherein $\sigma(t)$ is equal to $\mu_0(t)$ or a moving average filter of $\mu_0(t)$ with a specific sample window.

5. The method according to any of claims 1 to 4, further comprising a step of implementing a correction to $\mu_0$ as:

$$\mu_0(t) = \begin{cases} \max\big(-u_b, \mu_{dob}(t)\big) & if\ \mu_{fb}(t) > -u_b \\ \mu_{fb}(t) & if\ \mu_{fb}(t) \le -u_b \end{cases}$$

defining feedback actions ($\mu_{fb}$) and feedforward actions ($\mu_{ff}$) as:

$$\mu_{fb}(s) = K(s) \cdot \big(F_r(s) \cdot r(s) - y(s)\big)$$

$$\mu_{ff}(s) = K(s) \cdot y^*(s)$$

wherein $r(s)$ is a nominal value of incremental glucose with respect to $G_b$.

6. The method according to any of claims 1 to 5, wherein the controller $K(s)$ is defined as:

$$K(s) = \frac{k \cdot F(s) \cdot \tilde{G}_d^{-1}(s)}{1 + k \cdot F(s) \cdot \tilde{G}_d^{-1}(s) \cdot G_u(s)}$$

and the filters $F(s)$ and $F_r(s)$ are defined as:

$$F(s) = \frac{1}{(\alpha s + 1)^3}$$

$$F_r(s) = \frac{k_r}{(\alpha_r s + 1)^3}$$

wherein $k$, $k_r$, $\alpha$ and $\alpha_r$ are previously calculated parameters and r is a nominal value of incremental glucose with respect to $G_b$.

7. The method according to any of claims 1 to 6, wherein $\theta_{cr}$ is variable in time according to the conditions in which one counter-regulatory action is more favourable than the other.

8. The method according to any of claims 1 to 5, wherein rescue carbohydrates are administered as specific doses of a quantification level ($q_w$) calculated by means of:

$$\widetilde{w}(k) = \begin{cases} \left\lceil \left| \frac{z_w(k)}{q_w} \right| \right\rceil \cdot q_w & if\ z_w(k) > \underline{q}_w \wedge HypoFlag \\ 0 & otherwise \end{cases}$$

and wherein $\underline{q}_w$ is a minimum threshold of accumulated carbohydrates to activate a suggestion, $\underline{q}_w < q_w$ and:

$$HypoFlag = \tilde{G}(k) < \tilde{G}(k)_{thr} \vee G(k) < G(k)_{thr}$$

wherein $\tilde{G}(k)$ is a prediction for $G(k)$ in a defined time horizon $t_H$, $\tilde{G}(k)_{thr}$ and $G(k)_{thr}$ are predetermined thresholds for predicted glucose and measured glucose, respectively; and wherein:

$$z_w(k) = \widehat{w}(k) + \tau_s \cdot BW \cdot w(k)$$

$$\widehat{w}(k+1) = \begin{cases} 0 & if\ z_w(k) > \underline{q}_w \wedge \neg HypoFlag \\ \left(1 - \frac{\tau_s}{\tau_{cl}}\right) \cdot \big(z_w(k) - \widetilde{w}(k)\big) & otherwise \end{cases}$$

wherein *BW* is the body weight (kg) of the user, $\tau_s > 0$ is a sampling period and $\tau_{cl}$ is a clearance.

9. The method according to claim 8, wherein the prediction for *G(k)* is calculated as:

$$\tilde{G}(k) = G(k) + t_H \cdot \delta_G(k)$$

$\delta_G(k)$ being a discretisation of the filtered glucose derivative:

$$\delta_G(s) = \frac{s \cdot G(s)}{\tau_\delta \cdot s + 1}$$

wherein $\tau_\delta > \tau_s$ is a previously determined time constant.

10. The method according to any of claims 1 to 9, wherein glucagon is administered as specific doses of a quantification level ($q_v$) calculated by means of:

$$\tilde{v}(k) = \begin{cases} \left\lfloor \left| \frac{z_v(k)}{q_v} \right| \right\rfloor \cdot q_v & if\ z_v(k) > \underline{q_v} \wedge HypoFlag \\ 0\ otherwise \end{cases}$$

and wherein $\underline{q_v}$ is a minimum threshold of accumulated glucagon to activate a suggestion, $\underline{q_v} < q_v$ and:

$$HypoFlag = \tilde{G}(k) < \tilde{G}(k)_{thr} \vee G(k) < G(k)_{thr}$$

wherein $\tilde{G}(k)$ is a prediction for *G(k)* in a defined time horizon $t_H$, $\tilde{G}(k)_{thr}$ and $G(k)_{thr}$ are predetermined thresholds for predicted glucose and measured glucose, respectively; and wherein:

$$z_v(k) = \hat{v}(k) + \tau_s \cdot BW \cdot v(k)$$

$$\hat{v}(k+1) = \begin{cases} 0\ if\ z_v(k) > \underline{q_v} \wedge \neg HypoFlag \\ \left(1 - \frac{\tau_s}{\tau_{cl}}\right) \cdot \left(z_v(k) - \tilde{v}(k)\right) \qquad otherwise \end{cases}$$

wherein *BW* is the body weight (kg) of the user, $\tau_s > 0$ is a sampling period and $\tau_{cl}$ is a clearance.

11. The method according to claim 10, wherein the prediction for *G(k)* is calculated as:

$$\tilde{G}(k) = G(k) + t_H \cdot \delta_G(k)$$

$\delta_G(k)$ being a discretisation of the filtered glucose derivative

$$\delta_G(s) = \frac{s \cdot G(s)}{\tau_\delta \cdot s + 1}$$

wherein $\tau_\delta > \tau_s$ is a previously determined time constant.

12. The method according to any of claims 8 to 11, wherein $\tilde{G}(k)_{thr}$ is 60 mg/dl, $G(k)_{thr}$ is 54 mg/dl and the time horizon $t_H$ is 60 min.

13. The method according to any of claims 8 to 12, wherein the expected postprandial incremental plasma glucose $y^*(s)$ is calculated by adding the terms corresponding to specific doses of counter-regulatory actions, such as:

$$y^*(s) = G_u(s) \cdot u^*(s) + G_d(s) \cdot d^*(s) + G_v(s) \cdot \tilde{v}(s) \cdot \eta_v + G_w(s) \cdot \tilde{w}(s) \cdot \eta_w$$

wherein $\eta_v$ and $\eta_w$ are adjustable gains.

14. The method according to claim 13, wherein adjustable gains $\eta_v$ and $\eta_w$ are determined as the gains necessary to achieve the desired maximum output increase $y^*_{máx}$ when the counter-regulatory action (v, w) is equal to the quantification level ($q_v$, $q_w$), being obtained as:

$$\max\left(\mathcal{L}^{-1}\{q_v \cdot \eta_v \cdot G_v^j(s)\}\right) = y^*_{max}$$

$$\max\left(\mathcal{L}^{-1}\{q_w \cdot \eta_w \cdot G_w^j(s)\}\right) = y^*_{max}$$

wherein $\mathcal{L}^{-1}\{\cdot\}$ represents an inverse Laplace transform operator and $y^*_{máx}$ is the desired output variation due to the quantification level ($q_v$, $q_w$).

15. The method according to any of claims 1 to 14, wherein the expected postprandial incremental plasma glucose y*(s) is saturated as:

$$y^*_{sat}(t) = \begin{cases} y^*_{high} - G_b & if\ y^*(t) \geq y^*_{high} - G_b \\ y^*(t)\ if\ y^*_{high} - G_b > y^*(t) > y^*_{low} - G_b \\ y^*_{low} - G_b & if\ y^*(t) \leq y^*_{low} - G_b \end{cases}$$

16. The method according to claim 15, wherein $y^*_{high}$ is 180 and $y^*_{low}$ is 70.

17. The method according to any of claims 6 to 16, wherein an adjustable gain $k$ of the controller $K(s)$ is defined as $k = 0,65 \times 6 \times (1/CR^j)$.

18. The method according to any of claims 6 to 16, wherein the adjustable gain $k$ is defined as:

$$k = \min_{t>0} \frac{\mathcal{L}^{-1}\{G_d(s)\}(t) - \dfrac{y^{min}}{\tilde{M}}}{\mathcal{L}^{-1}\{F(s) \cdot G_u(s)\}(t - l_u)}$$

where $\mathcal{L}^{-1}$ is the inverse Laplace transform, and $y^{min}$ and $\tilde{M} > 0$ are adjustable parameters, with $\tilde{M}$ representing the expected meal size in the worst case, and $y^{min}$ the tolerable lower limit for the incremental postprandial glucose response to that meal $\tilde{M}$.

19. The method according to claim 18, wherein $\tilde{M}$ is defined as the size of the meal notified by the patient ($M^*$) whenever an optional meal notification is made, and $y^{min}$ is defined as the lower tolerable limit for the incremental postprandial glucose response to that meal $M^*$, maintaining the calculated value of k for an adjustable time window from the time the meal was notified.

20. The method according to any of claims 1 to 19, wherein the insulin bolus administered ($u^*$) as a result of an optional meal notification, is calculated from the size of the meal notified by the patient ($M^*$) as a bolus defined by $u^*(s) = C(s)M^*(s)$, wherein $C(s)$ is a feedforward controller given by:

$$C(s) = \frac{k_d}{k_u}$$

21. The method according to any of claims 1 to 19, wherein the insulin bolus administered ($u^*$) is calculated from the size of the meal notified by the patient ($M^*$) as a superbolus defined by $u^*(s) = C(s)M^*(s)$, wherein $C(s)$ is a feedforward controller given by:

$$C(s) = \frac{k_d}{k_u} + u_b \cdot T_\sigma - \frac{u_b}{s}(1 - e^{-T_\sigma s})$$

with $T_\sigma > 0$ being a time period in which the pump is turned off.

22. An artificial pancreas system for performing the method for controlling blood glucose according to any of claims 1 to 21, comprising:

    - a pump (3), to deliver insulin according to a coordinated control action ($u_T$) and/or an automated infusion of glucagon, according to coordinated control action ($v$, $\tilde{v}$);
    - a continuous glucose monitor (2) to measure the plasma glucose signal (G(t)); and
    - a first computing unit (1) configured to carry out the steps of any of claims 1 to 21.

23. The artificial pancreas system according to claim 22, further comprising a second pump to deliver the automated infusion of glucagon.

24. The artificial pancreas system according to any of claims 22 to 23, which further comprises a display to notify recommended control actions for the administration of glucagon and/or rescue carbohydrates.

25. The artificial pancreas system according to any of claims 22 to 24, further comprising a pen device for the patient to deliver the recommended glucagon control actions.

26. Computer program adapted to perform the steps of the method according to any of claims 1 to 21 using the defined computing unit of the artificial pancreas system according to any of claims 22 to 25.

27. A computer readable storage medium comprising the computer program according to claim 26.

**FIG. 1**

FIG. 2

EP 4 767 933 A1

**FIG. 3**

<div align="center">

**INTERNATIONAL SEARCH REPORT**

</div>

| | International application No. |
|---|---|
| | PCT/ES2024/070517 |

**A. CLASSIFICATION OF SUBJECT MATTER**

See extra sheet

According to International Patent Classification (IPC) or to both national classification and IPC

**B. FIELDS SEARCHED**

Minimum documentation searched (classification system followed by classification symbols)
A61B, A61M, G16H

Documentation searched other than minimum documentation to the extent that such documents are included in the fields searched

Electronic data base consulted during the international search (name of data base and, where practicable, search terms used)

EPODOC, INVENES, DWPI, NPL, INSPEC, BIOSIS, MEDLINE

**C. DOCUMENTS CONSIDERED TO BE RELEVANT**

| Category* | Citation of document, with indication, where appropriate, of the relevant passages | Relevant to claim No. |
|---|---|---|
| A | ES 2932844 A1 (UNIV. POLITÉCNICA VALENCIA)26/01/2023, page 4, line 13 - page 14, line 23; page 17, line 1 - page 30, line 18. | 1-27 |
| A | US 2022044783 A1 (BONDÍA, COMPAY ET AL.) 10/02/2022, paragraphs [20 - 59]; paragraphs [69 - 95]. | 1-27 |
| A | US 2022273874 A1 (BRETON ET AL.) 01/09/2022, paragraph [3]; paragraphs [10 - 13]; paragraphs [59 - 81]. | 1-27 |
| A | VINALS, CLARA et al.: "Artificial Pancreas with Carbohydrate Suggestion Performance for Unannounced and Announced Exercise in Type 1 Diabetes". Journal Of Clinical Endocrinology & Metabolism, 01/2021, Vol. 106, N° 1, pages 55-63. | 1-27 |

☒ Further documents are listed in the continuation of Box C.  ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
|---|---|
| 03/12/2024 | **(11/12/2024)** |

| Name and mailing address of the ISA/ | Authorized officer |
|---|---|
| OFICINA ESPAÑOLA DE PATENTES Y MARCAS Paseo de la Castellana, 75 - 28071 Madrid (España) Facsimile No.: 91 349 53 04 | A. Cárdenas Villar |
| | Telephone No. 913495393 |

Form PCT/ISA/210 (second sheet) (July 2022)

## INTERNATIONAL SEARCH REPORT

| International application No. |
| --- |
| PCT/ES2024/070517 |

**C (continuation).**                    DOCUMENTS CONSIDERED TO BE RELEVANT

| Category * | Citation of documents, with indication, where appropriate, of the relevant passages | Relevant to claim No. |
| --- | --- | --- |
| A | MOSCARDO, V. et al.: "In silico evaluation of a parallel control-based coordinated dual-hormone artificial pancreas with insulin on board limitation". 2019 American Control Conference (ACC), 12/07/2019, pages 4759 – 4764. | 1-27 |
| A | INFANTE, MARCO et al.: "Dual-hormone artificial pancreas for management of type 1 diabetes: Recent progress and future directions". Artificial Organs, 09/2021, Vol. 45, N° 9, pages 968-986. | 1-27 |
| A | ZENG, BAOQI: "Dual-hormone artificial pancreas for glucose control in type 1 diabetes: A meta-analysis". Diabetes, Obesity & Metabolism, 10/2022, Vol. 24, N° 10, pages 1967-1975. | 1-27 |
| A | HAIDAR, AHMAD: "A Novel Dual-Hormone Insulin-and-Pramlintide Artificial Pancreas for Type 1 Diabetes: A Randomized Controlled Crossover Trial". Diabetes Care, 03/2020, Vol. 43, N° 3, pages 597-606. | 1-27 |

Form PCT/ISA/210 (continuation of second sheet) (July 2022)

| INTERNATIONAL SEARCH REPORT | | International application No. | |
| --- | --- | --- | --- |
| Information on patent family members | | PCT/ES2024/070517 | |

| Patent document cited in the search report | Publication date | Patent family member(s) | Publication date |
| --- | --- | --- | --- |
| ES2932844 A1 | 26.01.2023 | WO2024023376 A1 | 01.02.2024 |
| US2022044783 A1 | 10.02.2022 | NONE | |
| US2022273874 A1 | 01.09.2022 | NONE | |

Form PCT/ISA/210 (patent family annex) (July 2022)

## INTERNATIONAL SEARCH REPORT

International application No.

PCT/ES2024/070517

CLASSIFICATION OF SUBJECT MATTER

*A61B5/145* (2006.01)
*A61M5/142* (2006.01)
*A61M5/172* (2006.01)
*G16H10/60* (2018.01)
*G16H40/60* (2018.01)